# EUROPEAN PATENT SPECIFICATION

(11) **EP 2 060 930 B1**
(45) Date of publication and mention of the grant of the patent: **17.10.2012**
(21) Application number: 08019896.3
(22) Date of filing: 14.11.2008
(51) Int. Cl.: G01S 15/89, A61B 8/08, G10K 11/34, G10K 11/35, A61B 8/15

(54) **Ultrasound diagnostic device having transducers facing each other**
Ultraschalldiagnosevorrichtung mit sich gegenüberliegenden Wandlern
Dispositif de diagnostic à ultrasons disposant de transducteurs se faisant face

(30) Priority: 14.11.2007 KR 20070116168
(43) Date of publication of application: 20.05.2009
(73) Proprietor: Samsung Medison Co., Ltd., Kangwon-do (KR)
(72) Inventor: Jeong, Mok Kun, Seoul 139-768 (KR); Kim, Yung Gil, Gangnam-gu Seoul 135-280 (KR); Kwon, Sung Jae, Gangnam-gu Seoul 135-505 (KR); Yoon, Ra Young, Gangnam-gu Seoul 135-280 (KR)
(74) Representative: Lorenz, Markus

(56) References cited:
- WO-A-02/089672
- DE-A1- 10 260 667
- US-A- 5 678 565
- US-A1- 2004 034 307
- US-A1- 2006 173 304

## Description

### BACKGROUND OF THE INVENTION

### [Technical Field]

The present invention generally relates to an ultrasound diagnostic device, and more particularly to an ultrasound diagnostic device having a pair of array transducers facing each other.

### [Background Art]

An ultrasound diagnostic device has become an important and popular diagnostic tool due to its non-invasive and non-destructive nature. The ultrasound diagnostic device may form an ultrasound image by utilizing wave characteristics of ultrasound such as reflection, scattering and absorption as ultrasound signals propagate through tissues of a human body. The ultrasound diagnostic device may have a transducer for transmitting ultrasound signals to a target object and receiving ultrasound echoes reflected back from it. An array transducer including a plurality of elements is widely used to obtain an enhanced ultrasound image.

Generally, as the ultrasound signals propagate through the target object, they are attenuated. Thus, it is difficult to obtain an ultrasound image having a high signal-to-noise ratio (SNR) for a relatively far area from the transducer.

US 2004/034307 A1 describes a method for increasing the speed of the parabolic marching method by about a factor of 256, which can be used to accomplish a number of objectives, e.g. to collect data to form true 3-D images or 3-D assembled from 2-D slices or to match the operating frequency of reflection tomography. US 2004/034307 A1 discloses in particular a device according to the preambule of claim 1.

US 2006/173304 A1 refers to an apparatus and related methods for obtaining volumetric ultrasound scans of a breast of a supine patient in which a fluid reservoir including a bottom flexible membrane contacts an upward-facing surface of the breast. The fluid reservoir is filled with water or other suitable acoustically conductive fluid until the bottom membrane covering the breast is submerged. A transducer surface of an ultrasound probe is submerged in the fluid and moved over and/or around the breast area to obtain the ultrasound scans.

WO 02/089672 describes a method of determining accurate values of acoustic parameters of tissues, comprising: transmitting first acoustic energy through the tissue along a path in a first direction; acquiring a first reflection signal generated by said tissue along said path from the transmitted first acoustic energy; transmitting second acoustic energy through the tissue along the path in a second direction opposite from the first direction; acquiring a second reflection signal generated by said tissue along said path from the transmitted second acoustic energy; and generating one or more of values of attenuation along the path, velocity of acoustic energy along the path, acoustic impedance of materials along the path and reflection along the path, responsive to both the first and second acquired reflection signals.

DE 102 60 667 A1 describes an Ultrasound unit, in which a woman's breast is compressed and fixed between two plates and ultrasound probes are moved over the plates in a computer controlled method in order to record an ultrasound image of the breast.

US-A-5 678 565 refers to a method and a device for noninvasively identifying a region of the tissue having a different elasticity than the surrounding tissue by simultaneously measuring strain and stress patterns in the tissue portion using an ultrasonic imaging system combined with a pressure sensing array.

### BRIEF DESCRIPTION OF THE DRAWINGS

FIG. 1 is a schematic diagram illustrating a pair of transducers.

FIGS. 2A and 2B are schematic diagrams showing examples of transmitting and receiving ultrasound signals by using two transducers.

FIGS. 3A to 3C are schematic diagrams illustrating examples of steering a plane wave used as an ultrasound transmit beam.

FIG. 4 is a schematic diagram showing an example of extension plates extended from both edges of the transducers.

FIGS. 5A and 5B are schematic diagrams showing examples of pressing a target object by using the transducers with extension plates mounted.

FIG. 6 is a schematic diagram showing elasticity images obtained under the condition that a target object is pressed.

FIG. 7 is a schematic diagram showing elasticity images with the motion of a target object compensated.

FIG. 8 is a block diagram showing an ultrasound diagnostic device in accordance with one embodiment of the present invention.

FIG. 9 is a block diagram showing an ultrasound diagnostic device in accordance with another embodiment of the present invention.

FIGS. 10A to 10C are schematic diagrams showing examples of obtaining ultrasound plane images by rotating the transducers.

FIG. 11 is a schematic diagram showing an example of rotating one of the transducers.

FIG. 12 is a schematic diagram illustrating an example of rotating one transducer around the other transducer.

FIG. 13 is a schematic diagram illustrating two pairs of transducers in accordance with further another embodiment.

### DETAILED DESCRIPTION OF THE INVENTION

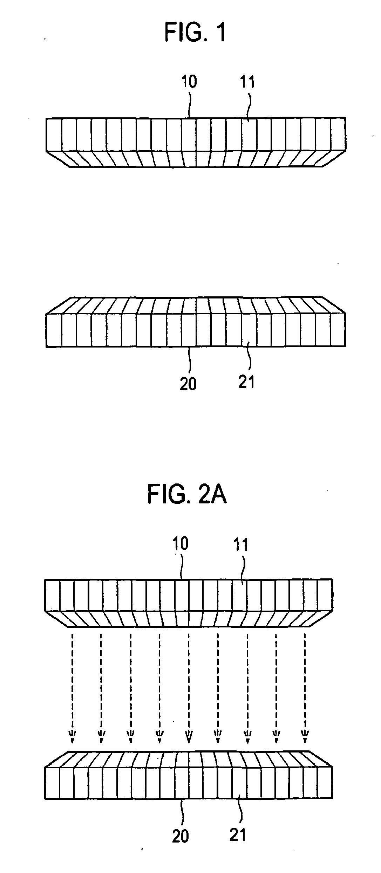

FIG. 1 is a schematic diagram showing an illustrative embodiment of a pair of transducers. As illustrated in FIG. 1, the ultrasound diagnostic device includes a pair of transducers 10 and 20. The pair of the transducers 10 and 20 are arranged to face each other. The transducers 10 and 20 may be set apart from each other by a predetermined distance.

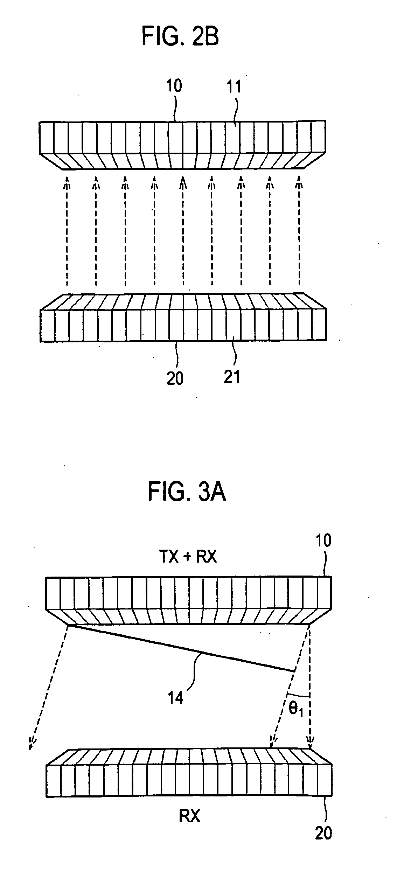

As illustrated in FIGS. 2A and 2B, each of the transducers 10 and 20 has an array transducer having a plurality of elements 11 and 21. The elements 11 and 21 may transmit the ultrasound signals to a target object and receive ultrasound echoes reflected from the target object to thereby output analog receive signals. The transducers 10 and 20 are a linear array transducer in which the elements are linearly arranged. A distance between two array transducers 10 and 20 is adjustable by moving at least one of the transducers 10 and 20 in an axial direction. The distance may be adjusted according to the type of a target object or diagnostic conditions. The target object may be positioned between two array transducers 10 and 20, so that an imaging error caused by a motion of the target object such as a human body may be reduced.

Since two array transducers 10 and 20 are arranged such that they face each other in one embodiment, images may be obtained in both reflection and transmission modes. That is, when one transducer emits ultrasound signals to a target object, the ultrasound signals may be reflected by the target object and received by the opposite transducer. The reflected ultrasound signals may be used to form a B-mode image in the reflection mode and the transmitted ultrasound signals may be used to form speed of sound and attenuation images in the transmission mode. Two array transducers 10 and 20 may alternately transmit an ultrasound transmit beam to the target object. In response to the transmission, each of two array transducers 10 and 20 may receive an ultrasound echo reflected from the target object in the reflection mode. With this configuration of two array transducers, it is possible to obtain two image frames corresponding to the same plane in the target object, each of which is formed based on the ultrasound echo. The two image frames may be compounded again by using a technique such as averaging. The so-obtained compound image frame may be presented as a new image frame.

In some embodiments, each of two array transducers 10 and 20 may be operable to transmit an ultrasound transmit beam several times at different steering angles. In this case, a plurality of image frames corresponding to the individual steering angles may be obtained based on the ultrasound echoes. The plurality of image frames may be compounded, so that two compound images corresponding to individual array transducers 10 and 20 may be obtained. Thereafter, the two compound images may be superposed again. Thus, speckles may be reduced through the compounding. Also, the degradation of resolution and SNR with increasing depth may be compensated for.

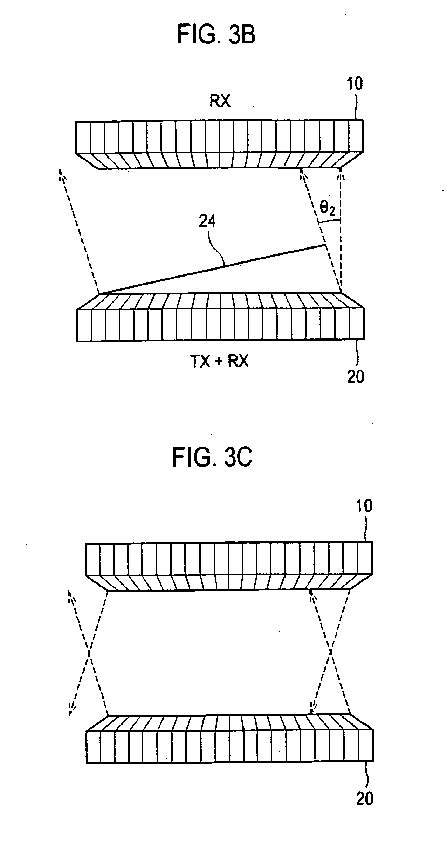

In the meantime, if the ultrasound transmit beam is transmitted by using a single element, the SNR may be decreased. A plane wave (e.g., a limited-diffraction beam) may be used as the ultrasound transmit beam to increase the SNR. The plane waves transmitted from the respective transducers 10 and 20 may be steered to obtain an ultrasound image which covers a large imaging area without physically or mechanically tilting the transducers 10 and 20. For example, if transmission is repeated in steering angle steps of 1° over a steering angle range of ±10°, a total of 21 frames may be obtained. Thus, it has an effect equivalent to tilting the transducer by 20°. An ultrasound image may be formed through synthetic focusing of receive signals obtained through the individual array transducers 10 and 20 by steering the plane wave. When the ultrasound transmit beams are transmitted after being steered at transmit angles of θ₁ and θ₂ from the individual array transducers 10 and 20, shadow regions, which are not scanned, may result from one of the steered ultrasound transmit beams. However, the shadow region may be compensated by the ultrasound transmit beam emitted from the other transducer, as illustrated in FIGS. 3A to 3C. That is, since a pair of transducers is used to obtain an image frame in one embodiment, the shadow region may be reduced when the steered transmit beams are adopted to get a wide field of view in imaging. Numeral references "14" and "24" in FIGS. 3A and 3B represent wave fronts of the plane waves used as the ultrasound transmit beams.

In the transmission mode, the receive signals may be obtained based on the ultrasound transmit beam emitted from the opposite transducer through the target object. In such a case, the speed of sound and attenuation may be measured based on the arrival time and amplitude of the ultrasound transmit beam emitted from one array transducer upon reception on the other array transducer. A tomography image showing the attenuation and the speed of the ultrasound signal in the target object may be obtained by using a tomographic reconstruction technique. The speed of sound may be 1450 m/s in normal tissues of the breast and 1550 m/s in the tumor, so that it may be determined whether or not the tumor exists through the speed of sound image. Also, the attenuation of the ultrasound signal in the tumor is greater than that in the normal tissue. Thus, the tumor may be diagnosed through the attenuation image. As mentioned above, the B-mode image may be obtained together with the tomography image in both the reflection and transmission modes in accordance with one embodiment, so that the examination time may be reduced.

In the meantime, the conventional ultrasound computerized tomography (CT) may be carried out by immersing an imaging object in water to obtain an ultrasound CT image such as an ultrasound breast image. Thus, ultrasound refraction may occur due to the water. However, since two transducers 10 and 20 are in direct contact with the target object in accordance with one embodiment, the refraction of the ultrasound signals may be reduced, so that an enhanced ultrasound image resolution may be obtained.

An ultrasound signal of 7 MHz, which is typically used to obtain a breast image, may be largely attenuated during propagation in the breast. Therefore, ultrasound echoes reflected from a relatively far region from the transducer may be very weak. However, since breast images may be obtained from each of both array transducers 10 and 20, and compounded to form a compound breast image in one embodiment, depth-dependent image characteristics such as the attenuation may be compensated for.

In order to form the compound ultrasound image, the bidirectional pixel based focusing may be used in addition to the synthetic focusing. Also, various focusing methods may be applied by using two transducers 10 and 20.

When the target object is positioned between two transducers 10 and 20, the distance between the transducers may be finely adjusted in accordance with one embodiment such that a pressure may be applied to the target object. That is, the pressure applied to the target object may be changed by adjusting the distance between two transducers 10 and 20. In such a case, the pressure applied to the target object may be in inverse proportion to the distance therebetween. Thus, the pressure applied to the target object may be indirectly computed by using the distance between the transducers 10 and 20 in accordance with one embodiment.

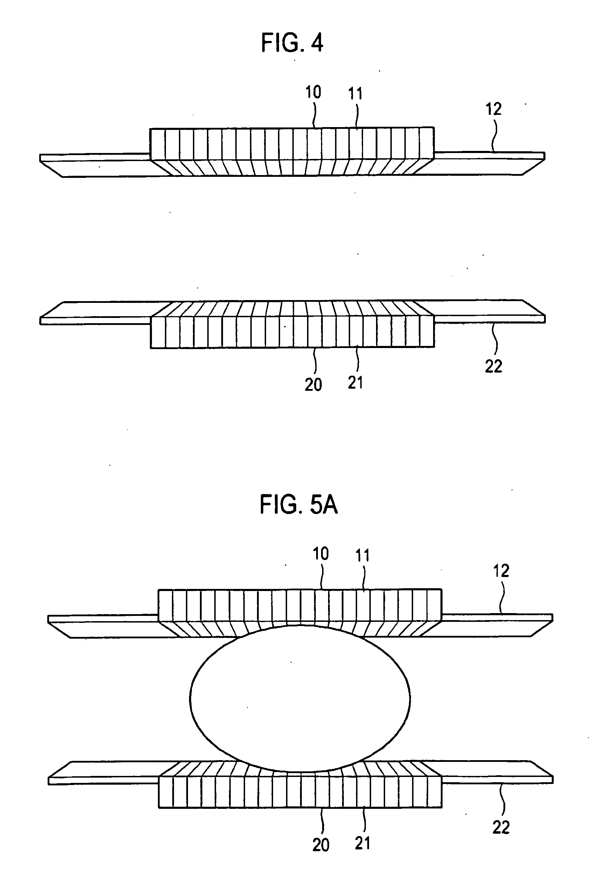

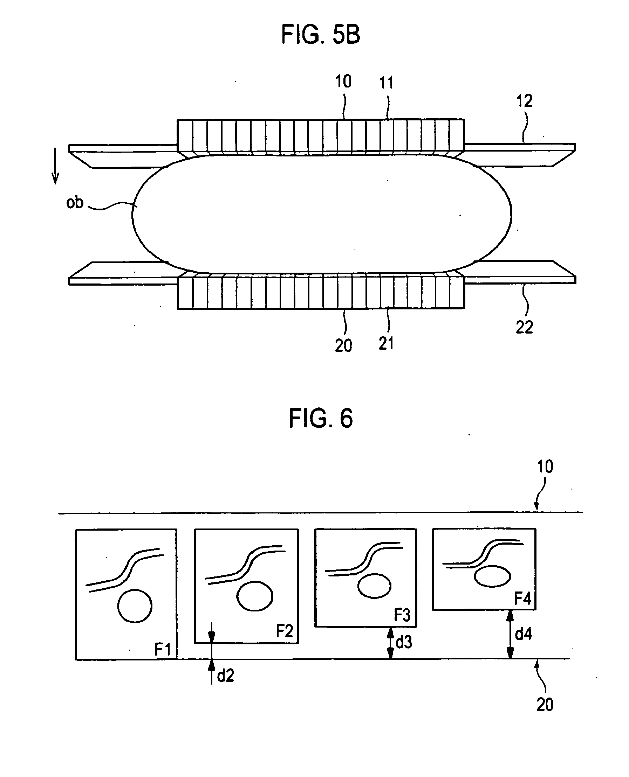

In accordance with another embodiment, the transducers 10 and 20 may include extension plates 12 and 22, which are extended from both edges of the transducers 10 and 20, respectively, as illustrated in FIG. 4. The extension plates 11 and 12 may be a compression plate for applying the pressure to the target object. When the pressure is applied to the target object Ob such as a breast to form the strain image, the pressure may be uniformly applied to the squeezed target object Ob through the compression plates 12 and 22, as illustrated in FIGS. 5A and 5B. Thus, a good strain image may be obtained compared with the strain image obtained by pressing the target object with palpation.

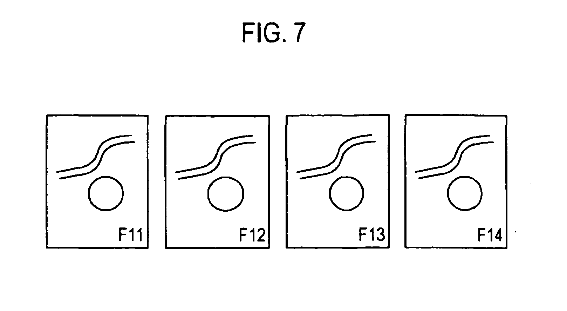

FIG. 6 is a schematic diagram showing examples of elasticity images (strain images) F1, F2, F3 and F4 obtained by pressing the target object in an axial direction of the transducers 10 and 20. FIG. 7 is a schematic diagram showing examples of elasticity images in which motions are compensated. The motion compensation may be performed by expanding the elasticity images F1, F2, F3 and F4 by a factor defined by a reciprocal number of the strain, so that the motion-compensated elasticity images F11, F12, F13 and F14 corresponding to different strains may be obtained. The elasticity images F11, F12, F13 and F14 may be averaged to form a final elasticity image. Thus, an error in strain calculation may be reduced through the averaging of the elasticity images. In accordance with one embodiment, two elasticity images may be obtained at every transmission and reception of the ultrasound signals from the two transducers 10 and 20 in the same manner of forming the B-mode images.

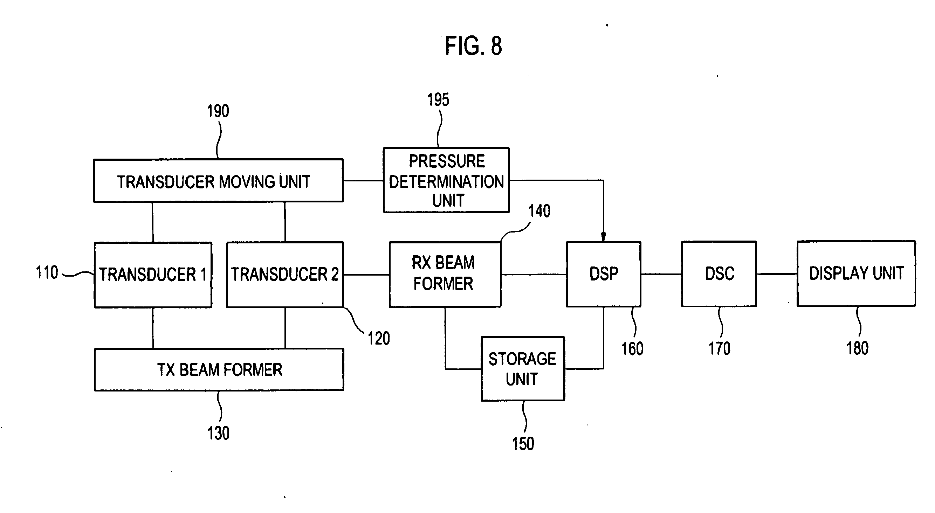

FIG. 8 is a block diagram showing an ultrasound diagnostic device including two array transducers 110 and 120. The two array transducers 110 and 120 are arranged to face each other. The array transducers 110 and 120 include a plurality of elements for transmitting ultrasound signals along scan lines, which are set in the target object in response to transmit pulse signals, and outputting analog receive signals based on ultrasound echoes reflected from the target object. A transmitting beamformer 130 is operable to apply delays to the transmit pulse signals such that the ultrasound signals are focused on the scan lines. A receive beamformer 140 is operable to sample the analog receive signals at a predetermined sampling rate to thereby form receive signals corresponding to the scan lines. The receive beamformer 140 includes an analog-to-digital converter operable to convert the analog receive signals to digital receive signals. The receive beamformer 140 may be operable to apply delays to the digital receive signals in consideration of a distance between the element and a focal point, and sum delayed digital receive signals to thereby form receive data. A storage unit 150 may store the receive data. A digital signal processor (DSP) 160 is operable to form image data corresponding to image modes such as a B-mode, a C-mode and a D-mode based on the receive data. A digital scan converter 170 may be operable to scan convert the image data to a data format suitable for display. A display unit 180 is operable to display an ultrasound image based on the scan-converted image data.

A moving unit 190, which is coupled to the transducers 110 and 120, is operable to move at least one of the transducers 110 and 120 in an axial direction. A pressure determination unit 195 is operable to determine a pressure applied to the target object based on a distance between the transducers 110 and 120. The DSP 160 is operable to form elasticity image data based on the pressure determined by the pressure determination unit 195. The digital scan converter 170 may be operable to scan convert the elasticity image data to an image format suitable for display. The display unit 180 displays an elasticity image based on the scan-converted elastic image data.

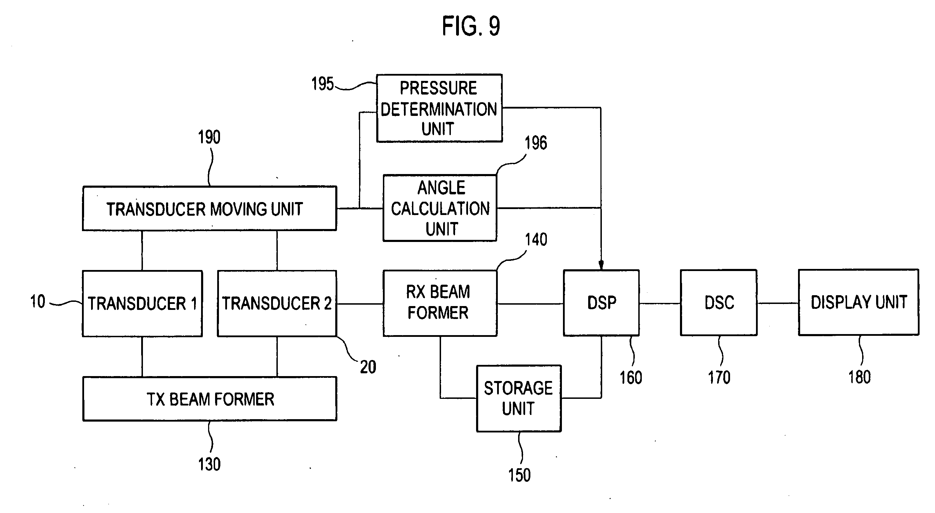

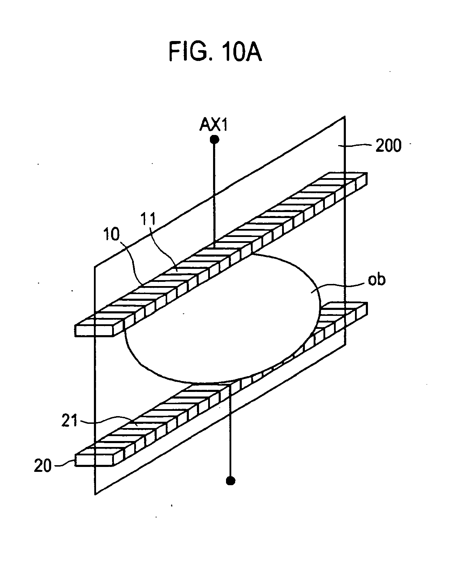

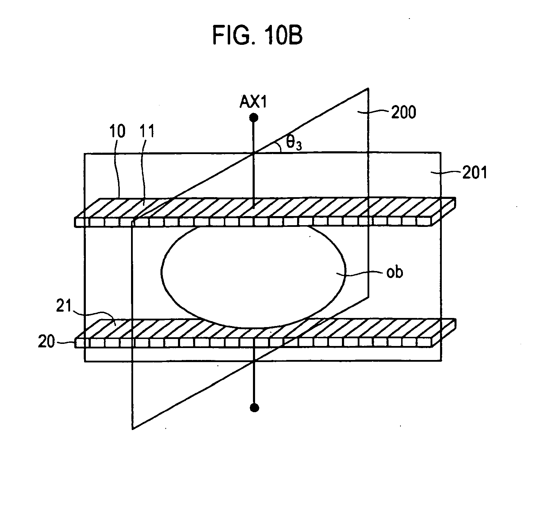

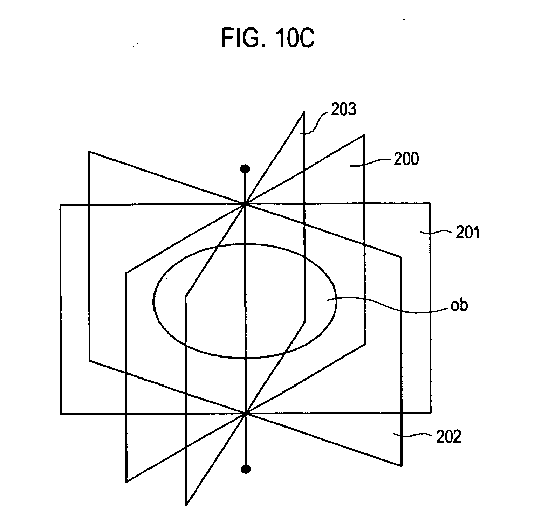

FIG. 9 is a block diagram showing an ultrasound diagnostic device including two array transducers 110 and 120 arranged to face each other in another embodiment. The ultrasound diagnostic device may further include a rotation angle calculation unit 196 in addition to all elements of the ultrasound diagnostic device illustrated in FIG. 8. The transducer moving unit 190 may be further operable to rotate the transducers 110 and 120 on the same planes, as illustrated in FIGS. 10A to 10C. The rotation angle calculation unit 196 may be operable to calculate a rotation angle of the array transducers 110 and 120.

Referring to FIGS. 10A to 10C, the transducers 10 and 20 may be rotated about a rotating axis AX₁, so that the DSP 160 may form a plurality of plane images 200, 201, 202 and 203. The DSP 160 may be further operable to synthesize the plane images in consideration of a rotation angle θ₃ to form a 3-dimensional image. The resolution of the 3-dimensional image may be determined by finely adjusting the rotation angle θ₃. The rotation angle θ₃ may be set to a small angle when a region of interest such as a cancer is scanned to get a high-resolution 3-dimensional ultrasound image for the region of interest.

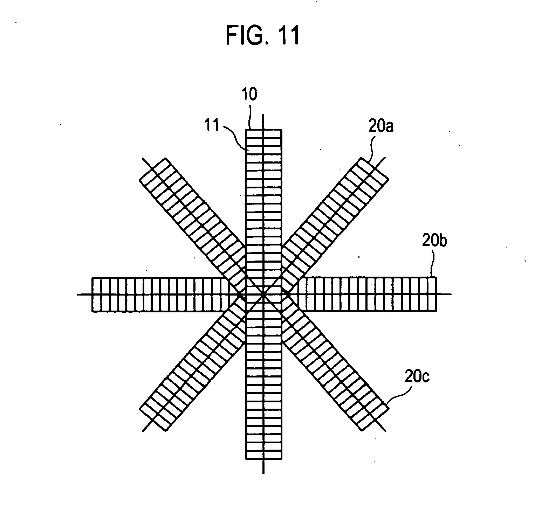

FIG. 11 is a schematic diagram showing an example of obtaining a plurality of ultrasound plane images by rotating only one transducer 20 on the same plane while the other transducer is fixed. Referring to FIG. 11, one of the transducers (e.g., transducer 20) may rotate to obtain a plurality of ultrasound plane images 20a, 20b and 20c. The DSP 160 may be operable to synthesize the ultrasound plane images 20a, 20b and 20c to thereby form a 3-dimensional ultrasound image.

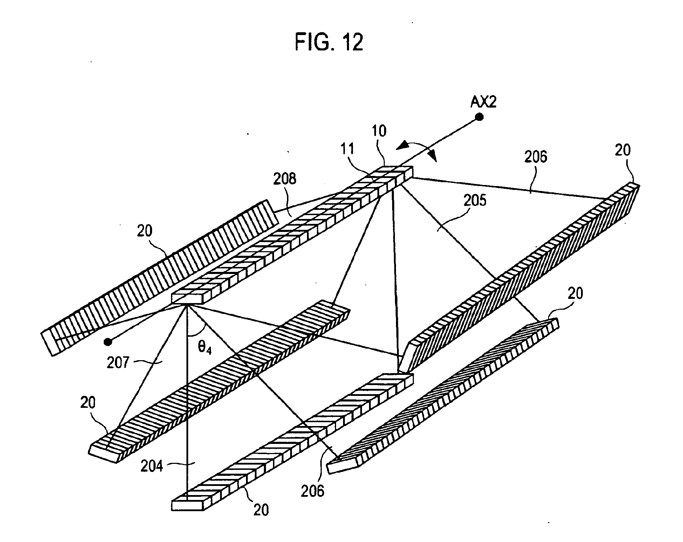

The transducer moving unit 190 may be further operable to rotate one transducer around the other transducer as illustrated in FIG. 12. In such a case, the moving transducer 20 may move along a surface of the target object while the two ultrasound transducers 10 and 20 face each other. That is, the transducer 10 is also rotated about a rotation axis AX₂ to face the transducer 20. Since the transducer 20 moves along the surface of the target object, a plurality of ultrasound plane images 204, 205, 206, 207 and 208 may be obtained. Thus, a wide region of the target object may be scanned. The DSP 160 may be operable to synthesize the ultrasound plane images 204, 205, 206, 207 and 208 in consideration of a rotation angle θ₄ to thereby form a 3-dimensional ultrasound image. A volume of internal tissues in the target object may be calculated by using the 3-dimensional ultrasound image.

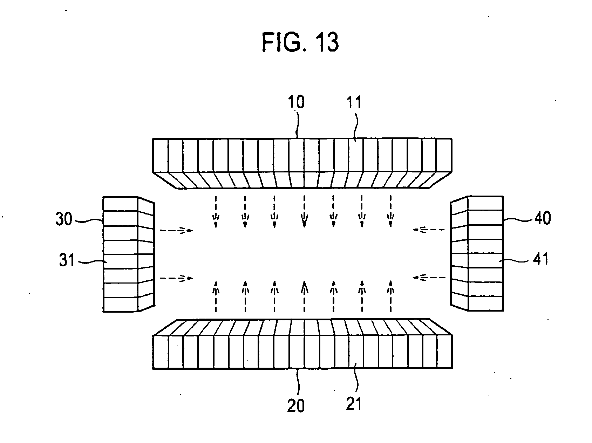

FIG. 13 is a schematic diagram illustrating two pairs of transducers in accordance with further another embodiment. As illustrating in FIG. 13, a pair of additional array transducers 30 and 40 may be further included in addition to the pair of array transducers 10 and 20. The array transducers 30 and 40 may be arranged such that they face each other similar to the array transducers 10 and 20. Each of the array transducers 30 and 40 may have a plurality of elements 31 and 41. Further, the array transducers 30 and 40 may be arranged to be perpendicular to the array transducers 10 and 20. Four images corresponding to the individual array transducers 10 to 40 may be obtained, and then compounded to form a compound image. Thus, an enhanced ultrasound image resolution may be obtained.

As mentioned above, the ultrasound diagnostic device of the present invention adopts two transducers facing each other, so that the amount of ultrasound image data that can be acquired may become twice that of the conventional ultrasound diagnostic device to form the ultrasound image. Thus, an enhanced ultrasound image may be formed.

In accordance with one embodiment of the present invention, there is provided an ultrasound diagnostic device, comprising a pair of transducers operable to transmit/receive ultrasound signals, wherein the transducers are arranged to face each other.

Any reference in this specification to "one embodiment," "an embodiment," "example embodiment," etc. means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to effect such a feature, structure or characteristic in connection with that of other embodiments.

Although embodiments have been described with reference to a number of illustrative embodiments thereof, it should be understood that numerous other modifications and embodiments can be devised by those skilled in the art that will fall within the scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound diagnostic device, comprising: a pair of transducers (10, 20) operable to transmit/receive ultrasound signals, wherein the transducers (10, 20) are arranged to face each other and each of the transducers (10, 20) is a linear array transducer including a plurality of elements;
a transmit beam former (130) operable to delay transmit pulse signals applied to the transducers (10, 20) such that the ultrasound signals are focused on scan lines set in a target object; a receive beam former (140) operable to convert analog receive signals outputted from the transducers in response to reception of ultrasound echoes reflected from the target object and delay the digital receive signals to form receive data; a digital signal processor (160) operable to form image data based on the receive data; a display unit (180) operable to display an ultrasound image based on the image data;
and a moving unit (190) operable to move at least one of the transducers (10, 20) in an axial direction to apply a pressure to the target object,
**characterized by** comprising a pressure determination unit (195) operable to determine the pressure applied to the target object based on a distance between the transducers, wherein the digital signal processing unit (160) is operable to form elasticity image data based on the digital receive data and the determined pressure, and the display unit (180) is operable to display an elasticity image based on the elasticity image data.

2. The ultrasound diagnostic device of Claim 1, further comprising compression plates (12, 22) extended from both edges of the respective transducers (10, 20).

3. The ultrasound diagnostic device of Claim 1, wherein the moving unit (190) is further operable to rotate at least one of the transducers (10, 20) about a rotation axis on a same plane, or one transducer around the other transducer while the transducers (10, 20) face each other.

4. The ultrasound diagnostic device of Claim 3, further comprising a rotation angle calculation unit (196) operable to calculate a rotation angle of the transducers (10, 20), wherein the digital signal processing unit (160) is further operable to form a plurality of plane images and form a 3-dimensional image by synthesizing the plane images based on the calculated rotation angle.

5. The ultrasound diagnostic device of Claim 1, further comprising a pair of additional transducers (30, 40), wherein the additional transducers (30, 40) are arranged to face each other and to be perpendicular to the transducers.

## Patentansprüche

1. Ultraschalldiagnosevorrichtung, welche Folgendes aufweist:
ein Paar Signalumformer (10,20), welche in der Lage sind, Ultraschallsignale zu übertragen bzw. zu empfangen, wobei die Signalumformer (10,20) so angeordnet sind, dass sie einander zugerichtet sind, und wobei jeder Signalumformer (10,20) ein Linearreihen-Signalumformer mit einer Vielzahl von Elementen ist,
einen Übertragungsstrahlformer (130), welcher in der Lage ist, auf die Signalumformer (10,20) angewendete Übertragungspulssignale so zu verzögern, dass die Ultraschallsignale auf in einem Zielobjekt festgelegte Scanlinien fokussiert werden; einen Empfangsstrahlformer (140), welcher in der Lage ist, von den Signalumformern als Realtion auf den Empfang von Ultraschallsignalen, die von dem Zielobjekt reflektiert wurden, ausgegebene analoge Empfangssignale zu konvertieren und die digitalen Empfangssignale zu verzögern, um Empfangsdaten zu bilden; einen digitalen Signalprozessor (160), welcher in der Lage ist, Bilddaten basierend auf den Empfangsdaten zu bilden; eine Anzeigeeinheit (180), welche in der Lage ist, ein Ultraschallbild basierend auf den Bilddaten anzuzeigen;
und eine Bewegungseinheit (190), welche in der Lage ist, wenigstens einen der Signalumformer (10,20) in einer axialen Richtung zu bewegen, um einen Druck auf das Zielobjekt aufzubringen,
**gekennzeichnet durch**
eine Druckermittlungseinheit (195), welche in der Lage ist, den auf das Zielobjekt aufgebrachten Druck basierend auf einem Abstand zwischen den Signalumformern zu ermitteln, wobei der Signalprozessor (160) in der Lage ist, Elastizitätsbilddaten basierend auf den digitalen Empfangsdaten und dem ermittelten Druck zu bilden, und wobei die Anzeigeeinheit (180) in der Lage ist, ein Elastizitätsbild basierend auf den Elastizitätsbilddaten anzuzeigen.

2. Ultraschalldiagnosevorrichtung nach Anspruch 1, welche des Weiteren Druckplatten (12,22) aufweist, die sich von beiden Rändern der entsprechenden Signalumformer (10,20) erstrecken.

3. Ultraschalldiagnosevorrichtung nach Anspruch 1, wobei die Bewegungseinheit (190) des Weiteren in der Lage ist, wenigstens einen der Signalumformer (10,20) um eine Rotationsachse auf einer gleichen Ebene oder einen Signalumformer um den anderen Signalumformer zu rotieren, während die Signalumformer (10,20) einander zugerichtet sind.

4. Ultraschalldiagnosevorrichtung nach Anspruch 3, welche des Weiteren eine Rotationswinkelberechnungseinheit (196) aufweist, welche in der Lage ist, einen Rotationswinkel der Signalumformer (10,20) zu berechnen, wobei der Signalprozessor (160) des Weiteren in der Lage ist, eine Vielzahl von Ebenenbilder zu bilden und ein 3-dimensionales Bild durch Synthetisieren der auf dem berechneten Rotationswinkel basierenden Ebenenbilder zu bilden.

5. Ultraschalldiagnosevorrichtung nach Anspruch 1, welche des Weiteren ein Paar zusätzlicher Signalumformer (30,40) aufweist, wobei die zusätzlichen Signalumformer (30,40) so angeordnet sind, dass sie einander zugerichtet sind und dass sie senkrecht zu den Signalumformern sind.

## Revendications

1. Dispositif de diagnostic ultrasonique comportant : une paire de transducteurs (10, 20) agencés pour émettre/recevoir des signaux ultrasoniques, dans lequel les transducteurs (110, 20) sont disposés en regard l'un de l'autre et chacun des transducteurs (10, 20) est un transducteur à réseau linéaire comportant une pluralité d'éléments ;
un générateur de rayon de transmission (130) agencé pour transmettre de façon temporisée des signaux pulsés appliqués aux transducteurs (10, 20) tels que les signaux ultrasoniques sont focalisés sur des lignes de balayage d'un objet cible; un récepteur de rayon (140) agencé pour convertir des signaux analogiques émis par les transducteurs en réponse à la réception d'échos ultrasoniques réfléchis par l'objet cible et temporiser les signaux numériques reçus pour former des données réceptionnées; une unité de traitement de signal numérique (160) agencée pour former une donnée image basée sur les données enregistrées; une unité d'affichage (180) agencée pour afficher l'image ultrasonique basée sur la donnée image;
et une unité mobile (190) agencée pour déplacer au moins un des transducteurs (10, 20) dans une direction axiale pour appliquer une pression sur l'objet cible,
**caractérisé en ce qu'**il comprend une unité de détermination de la pression (195) agencée pour déterminer la pression appliquées sur l'objet cible, basée sur la distance entre les deux transducteurs, dans lequel l'unité de traitement du signal numérique (160) est agencée pour former une donnée image déformable basée sur le signal reçu et de déterminer la pression, et l'unité d'affichage (180) est agencée pour afficher une image déformable basée sur la donnée d'image déformable.

2. Dispositif de diagnostic ultrasonique selon la revendication 1, comportant en outre des plaques de compression (12, 22) qui s'étendent à partir des deux bords des transducteurs respectifs (10, 20).

3. Dispositif de diagnostic ultrasonique selon la revendication 1, dans lequel l'unité mobile (190) est en outre agencée pour tourner au moins l'un des transducteurs (10, 20) autour d'un axe de rotation sur un même plan, ou un des transducteurs autour de l'autre transducteur alors que les transducteurs (10, 20) sont disposés en regard l'un de l'autre.

4. Dispositif de diagnostic ultrasonique selon la revendication 3, comprenant en outre une unité de calcul de l'angle de rotation (196) agencée pour calculer l'angle de rotation des transducteurs (10, 20), dans lequel l'unité de traitement de signal numérique (160) est en outre agencée pour former une pluralité d'images planes et de former une image en 3-dimensions par synthèse des images planes en se basant sur le calcul de l'angle de rotation.

5. Dispositif de diagnostic ultrasonique selon la revendication 1, comprenant en outre une paire de transducteurs additionnels (30, 40) dans lequel les transducteurs additionnels (30, 40) sont disposés face à face et sont perpendiculaires aux transducteurs.
